# EUROPEAN PATENT APPLICATION

(11) **EP 3 360 475 A1**
(43) Date of publication of application: **15.08.2018**
(21) Application number: 17191199.3
(22) Date of filing: 14.09.2017
(51) Int. Cl.: A61B 5/0452, A61B 5/00, A61B 5/021

(54) **DEVICE FOR MEASURING BLOOD PRESSURE AND METHOD FOR USING THE SAME**

(30) Priority: 14.02.2017 CN 201710078974
(71) Applicant: Rooti Labs Limited, 1-1112 Grand Cayman (KY)
(72) Inventor: LI, Ko-Mai, Taipei City, Taiwan (CN)
(74) Representative: CMS Cameron McKenna Nabarro Olswang LLP

(57) **Abstract**

A device for measuring blood measure measures variations in electrical charges on the body as a result of heart activity. A method using the same is also described. The method acquires ECG signal collected by a collection unit of the device for measuring blood measure, processes the ECG signal, extracts feature from the ECG signal, and computes blood pressure according to the feature extracted from the ECG signal.

## Description

### 1. Technical Field

The invention relates to health, especially relates to a device for measuring blood measure and a method using the same.

### 2. Description of Related Art

A conventional sphygmomanometer includes cuff sphygmomanometer and invasive sphygmomanometer. However, the cuff sphygmomanometer may discomfort users, and the invasive sphygmomanometer is inconvenient for using.

Therefore, it is desirable to provide a device for measuring blood measure and a method using the same to overcome the described limitations.

### BRIEF DESCRIPTION OF THE DRAWINGS

Many aspects of the disclosure can be better understood with reference to the following drawings. The components in the drawings are not necessarily drawn to scale, the emphasis instead being placed upon clearly illustrating the principles of the disclosure. Moreover, in the drawings, like reference numerals designate corresponding parts throughout the several views.
FIG. 1 is a block diagram of an embodiment of a device for measuring blood pressure.
FIG. 2 is a time-domain schematic diagram of an embodiment of an electrocardiographic signal.
FIG. 3 is a block diagram of an embodiment of a system for measuring blood pressure.
FIG. 4 is a flowchart of an embodiment of a method for measuring blood pressure.

### DETAILED DESCRIPTION

It will be appreciated that for simplicity and clarity of illustration, where appropriate, reference numerals have been repeated among the different figures to indicate corresponding or analogous elements. In addition, numerous specific details are set forth in order to provide a thorough understanding of the embodiments described herein. However, it will be understood by those of ordinary skill in the art that the embodiments described herein can be practiced without these specific details. In other instances, methods, procedures, and components have not been described in detail so as not to obscure the related relevant feature being described. Also, the description is not to be considered as limiting the scope of the embodiments described herein. The drawings are not necessarily to scale and the proportions of certain parts may be exaggerated to better illustrate details and features of the present disclosure.

The present disclosure, including the accompanying drawings, is illustrated by way of examples and not by way of limitation. Several definitions that apply throughout this disclosure will now be presented. It should be noted that references to "an" or "one" embodiment in this disclosure are not necessarily to the same embodiment, and such references mean "at least one."

The term "module", as used herein, refers to logic embodied in hardware or firmware, or to a collection of software instructions, written in a programming language, such as, Java, C, or assembly. One or more software instructions in the modules can be embedded in firmware, such as in an EPROM. The modules described herein can be implemented as either software and/or hardware modules and can be stored in any type of non-transitory computer-readable medium or other storage device. Some non-limiting examples of non-transitory computer-readable media include CDs, DVDs, BLU-RAY, flash memory, and hard disk drives. The term "comprising" means "including, but not necessarily limited to"; it specifically indicates open-ended inclusion or membership in a so-described combination, group, series, and the like.

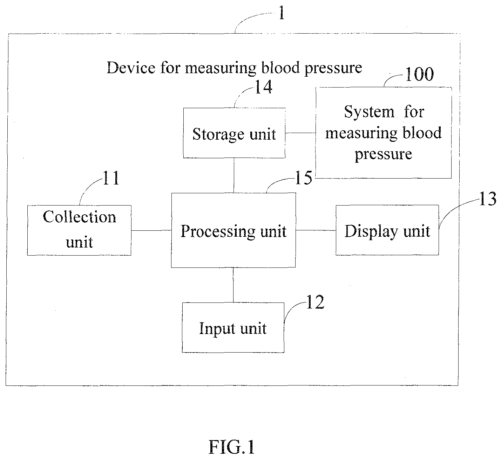

FIG. 1 illustrates an embodiment of a device 1 for measuring blood pressure. The device 1 includes a collection unit 11, an input unit 12, a display unit 13, a storage unit 14, and a processing unit 15. The collection unit 11 is configured to collect electrocardiogram (ECG) signal, for example human body's electrocardiogram signal. In at least one embodiment, the collection unit 11 includes two electrodes. The EGC signal reflects when the cardiac is in between systolic state and diastolic state. Cardiac muscle has a spontaneous beat and rhythmic contractions. A radio wave sent by a cardiac conduction system can irritate the cardiac muscle fibers to make the cardiac muscle contract. Accordingly, when the cardiac conduction system generates and sends the radio wave, the cardiac muscle produces a weak current distribution to human body. When the electrodes of the collection unit 11 are connected to different parts of human body, an electrocardiogram is depicted via the collection unit 11. It is well known that, when excited, the myocardial cells will produce a cardiac change between contraction and relaxation will happen. With the periodic cardiac change between contraction and relaxation happens, the pressure of artery in human body fluctuates. Thereby, blood pressure information can be obtained from the ECG signal.

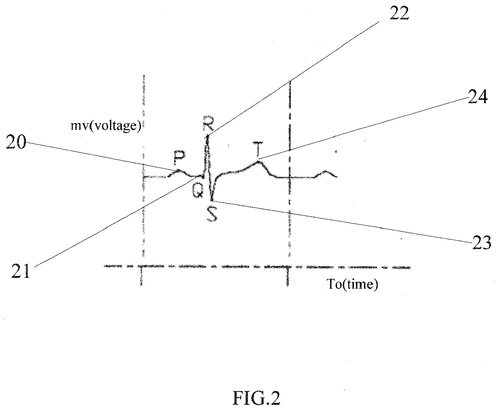

FIG. 2 illustrates a time-domain schematic diagram of an embodiment of ECG signal. The ECG signal indicates cardiac pulse wave. The ECG signal includes P wave, QRS complex wave, and T wave. Physiological significance of each wave of the ECG signal is that the P wave represents atria depolarization, QRS complex wave represents ventricular depolarization, and the T wave represents ventricle repolarization. The PR interval is a time period between when atria depolarizes and when depolarization wave generated by atria depolarization is conducted to atrioventricular node. The QT interval is a time period between when ventricle depolarizes and when ventricle repolarizes. The PR interval of the cardiac pulse wave is the systolic period of cardiac activity, and the PST interval of the cardiac pulse wave is the diastolic period of cardiac activity.

The input unit 12 is configured for user input. In at least one embodiment, the input unit 12 can be keyboard, touch screen, or combination of the keyboard and the touch screen. The display unit 13 is configured to display data of the device 1. In at least one embodiment, the display unit 13 can be a liquid crystal display or an organic light-emitting display. In at least one embodiment, the storage unit 14 can include various types of non-transitory computer-readable storage mediums. For example, the storage unit 14 can be an internal storage system, such as a flash memory, a random access memory (RAM) for temporary storage of information, and/or a read-only memory (ROM) for permanent storage of information. The storage unit 14 can also be an external storage system, such as a hard disk, a storage card, or a data storage medium. The processing unit 15 can be a central processing unit (CPU), a microprocessor, or other data processor chip. In at least one embodiment, the processing unit 15 performs functions of a system 100 for measuring blood pressure.

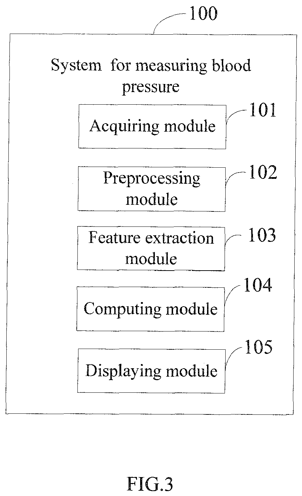

FIG. 3 illustrates an embodiment of the system 100 for measuring blood pressure. In at least one embodiment, the system 100 includes an acquiring module 101, a preprocessing module 102, a feature extraction module 103, a computing module 104, and a displaying module 105. The modules 101-105 of the system 100 can be collections of software instructions stored in the storage unit 14 of the device 1 and executed by the processing unit 15 of the device 1. The modules 101-105 of the system 100 also can include functionality represented as hardware or integrated circuits, or as software and hardware combinations, such as a special-purpose processor or a general-purpose processor with special-purpose firmware.

The acquiring module 101 acquires the ECG signal collected by the collection unit 11. In at least one embodiment, the collection unit 11 is an electrocardiogram recorder, the acquiring module 101 is used to acquire the ECG signal collected by the collection unit 11.

The preprocessing module 102 processes the ECG signal. In at least one embodiment, the preprocessing module 102 amplifies and filters the ECG signal. For example, the preprocessing module 102 amplifies the ECG signal and filters the amplified ECG signal to remove high frequency components of the ECG signal via an FIR low-frequency filter. In at least one embodiment, the preprocessing module 102 further converts the amplified ECG signal from analog to digital form.

The feature extraction module 103 extracts feature from the ECG signal. In at least one embodiment, the feature of the ECG signal includes time domain feature, frequency domain feature, linear feature, and non-linear feature. The time domain feature includes amplitude of the ECG signal and time interval of the ECG signal. The frequency domain feature includes various frequency components. The linear feature includes heart rate variability. Referring to FIG. 2, the feature extraction module 103 extracts P wave top 20, Q wave bottom 21, R wave top 22, S wave bottom 23, and T wave top 24 from the time domain feature of the ECG signal. The feature extraction module 103 further determines the systolic period S1 and the diastolic period S2 according to the P wave top 20, Q wave bottom 21, R wave top 22, S wave bottom 23, and T wave top 24. In at least one embodiment, the feature extraction module 103 extracts the P wave top 20, Q wave bottom 21, R wave top 22, S wave bottom 23, and T wave top 24 from the ECG signal, according to a differential threshold algorithm. The feature extraction module 103 determines an interval between the P wave top 20 and the R wave top 22 as the systolic period S1, and determines an interval between the P wave top 20 and the T wave top 24 as the diastolic period S2.

The computing module 104 computes blood pressure according to the feature extracted from the ECG signal. In at least one embodiment, the computing module 104 determines blood pressure according to the extracted feature and a relation table defining relationship between feature and blood pressure. In one embodiment, the feature of ECG signal recorded by an electrocardiogram recorder is associated with blood pressure recorded by a sphygmomanometer to form the relation table. The relation table is stored in the storage unit 14. In at least one embodiment, the computing module 104 processes the feature of the ECG signal according to linear regression algorithm and logistic regression algorithm, and determines blood pressure according to the processed feature of the ECG signal and the relation table.

In another embodiment, the computing module 104 computes blood pressure according to the systolic period S1 and the diastolic period S2. The blood pressure includes systolic pressure or diastolic pressure. In at least one embodiment, the computing module 104 computes blood pressure according to a first formula *P = M* × *F* + C. Wherein, M, and C are preset parameters related to blood pressure, F can be computed according to a second formula *F* = 1 / S2 , and S2 is the diastolic period extracted by the feature extraction module 103. The preset parameters M and C relate to the blood pressure.

In at least one embodiment, the preset parameters can be input by a user. In one embodiment, the computing module 104 first determines an empirical value as parameter value of C. The computing module 104 then receives a measuring pressure P measured by a sphygmomanometer and input by the user and computes the M based on a third formula *M* =(*P-C*)/*F.* Finally, the computing module 104 computes the blood pressure according to the first formula *P = M* × *F* + *C .*

In at least one embodiment, the displaying module 105 displays the computed blood pressure on the displaying unit 13.

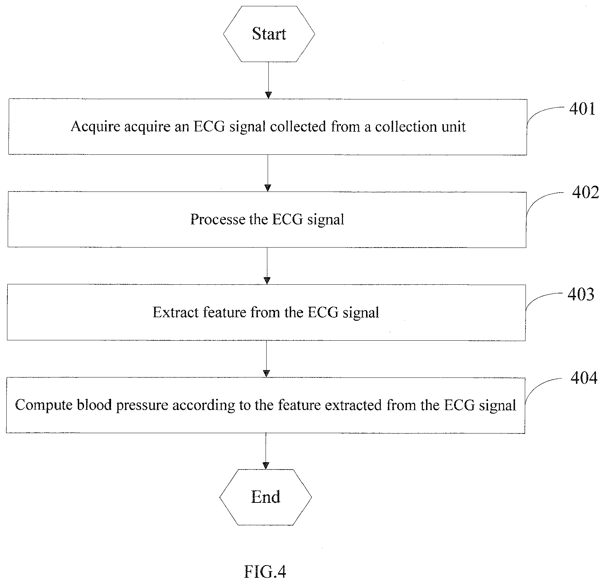

FIG. 4 illustrates a flowchart of a method for measuring blood pressure. The method is run in a device for measuring blood pressure. The method is provided by way of example, as there are a variety of ways to carry out the method. The method described below can be carried out using the configurations illustrated in FIGS. 1-3, for example, and various elements of these figures are referenced in explaining the example method. Each block shown in FIG. 4 represents one or more processes, methods, or subroutines carried out in the example method. Furthermore, the illustrated order of blocks is by example only and the order of the blocks can be changed. Additional blocks may be added or fewer blocks may be utilized, without departing from this disclosure. The example method can begin at block 401.

At block 401, a device for measuring blood pressure acquires an ECG signal collected from a collection unit. In at least one embodiment, the collection unit is a electrocardiogram recorder.

At block 402, the device or measuring blood pressure processes the ECG signal. In at least one embodiment, the device for measuring blood pressure amplifies and filters the ECG signal. For example, the device for measuring blood pressure amplifies the ECG signal and filters the amplified ECG signal to remove high frequency components of the ECG signal via an FIR low-frequency filter. The device for measuring blood pressure further converts the amplified ECG signal from analog to digital form.

At block 403, the device for measuring blood pressure extracts feature from the ECG signal. In at least one embodiment, the device for measuring blood pressure extracts P wave top, Q wave bottom, R wave top, S wave bottom, and T wave top from the time domain feature of the ECG signal. The device for measuring blood pressure further determines the systolic period S1 and the diastolic period S2 according to the P wave top, Q wave bottom, R wave top, S wave bottom, and T wave top. In at least one embodiment, the device for measuring blood pressure does differential threshold algorithm to the ECG signal to extract the P wave top, Q wave bottom, R wave top, S wave bottom and T wave top. The device for measuring blood pressure further determines the interval between the P wave top and the R wave top as the systolic period S1, and determines the interval between the P wave top and the T wave top as the diastolic period S2.

At block 404, the device for measuring blood pressure computes blood pressure according to the feature extracted from the ECG signal. In at least one embodiment, the device for measuring blood pressure determines blood pressure according to the extracted feature and a relation table defining relationship between the feature and blood pressure. In one embodiment, the feature of ECG signal recorded by an electrocardiogram recorder is associated with blood pressure recorded by a sphygmomanometer to form the relation table. The relation table is stored in a storage unit 14. In at least one embodiment, the device for measuring blood pressure processes the feature of the ECG signal according to linear regression algorithm and logistic regression algorithm, and determines blood pressure according to the processed feature of the ECG signal and the relation table.

In another embodiment, the device for measuring blood pressure computes blood pressure according to the systolic period S1 and the diastolic period S2. In at least one embodiment, the device for measuring blood pressure computes blood pressure based on a first formula *P = M* × *F* + C. Wherein, M, and C are preset parameters related to blood pressure, F can be computed based on a second formula *F* =1/*S*2.

In at least one embodiment, the preset parameters can be input by a user. In one embodiment, the device for measuring blood pressure first determines an empirical value as parameter value of C. the device for measuring blood pressure then receives a measuring pressure P measured by a sphygmomanometer and input by the user and computes the M based on a third formula *M* = (*P - C*)/*F.* Finally, the device for measuring blood pressure computes the blood pressure according to the first formula *P = M* × *F* + *C .*

In the embodiment, the method further includes: display the computed blood pressure on a displaying unit for a user to view.

Although the present disclosure has been specifically described on the basis of the exemplary embodiment thereof, the disclosure is not to be construed as being limited thereto. Various changes or modifications may be made to the embodiment without departing from the scope and spirit of the disclosure.

## Claims

1. A device for measuring blood measure, comprising:
a collection unit configured to collect electrocardiogram (ECG) signal;
a processing unit coupled to the collection unit;
a non-transitory storage medium coupled to the processing unit and configured to store a plurality of instructions which cause the device to:
acquire the ECG signal collected by the collection unit;
process the ECG signal;
extract feature from the ECG signal; and
compute blood pressure according to the feature extracted from the ECG signal.

2. The device according to the claim 1, wherein the plurality of instructions is further configured to cause the device to:
amplify and filter the ECG signal.

3. The device according to the claim 2, wherein the plurality of instructions is further configured to cause the device to:
amplify the ECG signal and filter the amplified ECG signal to remove high frequency components of the ECG signal via an FIR low-frequency filter.

4. The device according to the claim 1, wherein the feature of the ECG signal comprises time domain feature, frequency domain feature, linear feature and non-linear feature.

5. The device according to the claim 4, wherein the plurality of instructions is further configured to cause the device to:
extract P wave top, Q wave bottom, R wave top, S wave bottom, and T wave top from the time domain feature of the ECG signal.

6. The device according to the claim 5, wherein the plurality of instructions is further configured to cause the device to:
determine the blood pressure according to the extracted feature and a relation table defining relationship between the feature and the blood pressure.

7. The device according to the claim 6, wherein the plurality of instructions is further configured to cause the device to:
process the feature of the ECG signal according to a linear regression algorithm and a logistic regression algorithm; and
determine the blood pressure according to the processed feature of the ECG signal and a relation table defining relationship between the feature and the blood pressure.

8. The device according to the claim 5, wherein the plurality of instructions is further configured to cause the device to:
compute the blood pressure according to a first formula *P = M* × *F* + C, wherein, M, and C are preset parameters related to the blood pressure, F can be computed according to a second formula *F* =1/*S*2, S2 is a diastolic period between the P wave top and the T wave top.

9. The device according to the claim 8, wherein the plurality of instructions is further configured to cause the device to:
determine an empirical value as parameter value of C;
receive a measuring pressure P measured by a sphygmomanometer and input by an input unit;
compute the M based on a third formula *M* = (*P -* C) / *F ;* and
compute the blood pressure according to the first formula *P = M* × *F* + *C .*

10. A method for measuring blood pressure, applied on a device for measuring blood measure, the method comprising;
acquiring ECG signal collected by a collection unit of the device for measuring blood measure;
processing the ECG signal;
extracting feature from the ECG signal; and
computing blood pressure according to the feature extracted from the ECG signal.

11. The method according to claim 10, further comprising:
extracting P wave top, Q wave bottom, R wave top, S wave bottom, and T wave top from the time domain feature of the ECG signal.

12. The method according to claim 11, further comprising:
determine the blood pressure according to the extracted feature and a relation table defining relationship between the feature and the blood pressure.

13. The method according to claim 12, further comprising:
processing the feature of the ECG signal according to a linear regression algorithm and a logistic regression algorithm; and
determining the blood pressure according to the processed feature of the ECG signal and a relation table defining relationship between the feature and the blood pressure.

14. The method according to claim 11, further comprising:
compute the blood pressure according to a first formula *P = M* × *F* + C, wherein, M, and C are preset parameters related to the blood pressure, F can be computed according to a second formula *F* = 1/*S*2, S2 is a diastolic period between the P wave top and the T wave top.

15. The method according to claim 14, further comprising:
determining an empirical value as parameter value of C;
receiving a measuring pressure P measured by a sphygmomanometer and input by an input unit;
computing the M based on a third formula *M* = (*P-C)* / *F ;* and
computing the blood pressure according to the first formula *P* = *M* × *F* + *C.*
